Europäisches Patentamt

European Patent Office

Office européen des brevets

(11) Veröffentlichungsnummer: **0 001 403**
A1

# EUROPÄISCHE PATENTANMELDUNG

(21) Anmeldenummer: 78100872.7

(22) Anmeldetag: 12.09.78

(51) Int. Cl.²: **A 61 B 1/06, F 21 M 1/00**

(30) Priorität: 30.09.77 DE 2744140

(43) Veröffentlichungstag der Anmeldung: 18.04.79
Patentblatt 79/8

(84) Benannte Vertragsstaaten: **DE FR GB**

(71) Anmelder: SIEMENS AKTIENGESELLSCHAFT Berlin und München, Postfach 261, D-8000 München 22 (DE)

(72) Erfinder: Behringer, Wolfgang, Henri-Dunant-Strasse 36, D-6140 Bensheim (DE)
Erfinder: Heubeck, Erich, Blütenweg 11, D-6140 Bensheim (DE)

(54) Beleuchtungsvorrichtung für ärztliche, insbesondere zahnärztliche Zwecke.

(57) Die Erfindung bezieht sich auf eine Beleuchtungsvorrichtung mit einem Reflektor (2) und einer vorzugsweise in dessen innerem Brennpunkt angeordneten Lichtquelle (4), welche außerhalb des Reflektors, vorzugsweise in dessen äußerem Brennpunkt, einen relativ eng begrenzten Lichtfleck erzeugen. Zur Änderung der Helligkeit des Lichtfleckes ist erfindungsgemäß im unmittelbaren Bereich der Lampe (4) zwischen dieser (4) und dem Reflektor (2) in den Strahlengang des Lichtes eine die Strahlen wenigstens teilweise abschirmende Blende (9) angeordnet. Die Blende ist außerdem im Sinne einer Abschirmung der Strahlen vom Zentrum zur Peripherie des Reflektors hin verstellbar angeordnet.

Die Beleuchtungsvorrichtung ist für ärztliche, insbesondere zahnärztliche Zwecke vorgesehen.

EP 0 001 403 A1

SIEMENS AKTIENGESELLSCHAFT     Unser Zeichen
Berlin und München             VPA 77 P 5116 EPC

Beleuchtungsvorrichtung für ärztliche, insbesondere zahnärztliche Zwecke

Die Erfindung bezieht sich auf eine Beleuchtungsvorrichtung für ärztliche, insbesondere zahnärztliche Zwecke, mit einem Reflektor und einer vorzugsweise in dessen innerem Brennpunkt angeordneten Lichtquelle, welche außerhalb des Reflektors, vorzugsweise in dessen äußerem Brennpunkt, einen relativ eng begrenzten Lichtfleck erzeugt, sowie mit Mitteln zur Änderung der Helligkeit des Lichtfleckes.

Bei Beleuchtungsvorrichtungen dieser Art, die vorwiegend zum Ausleuchten relativ kleiner Körperpartien, z.B. zum Ausleuchten der Mundhöhle eines Patienten, dienen und deshalb in einer relativ kurzen Entfernung von der Lichtquelle einen relativ eng begrenzten Lichtfleck von etwa 100 x 200 mm Größe erzeugen, ist eine Änderung der Helligkeit zur Anpassung an die auszuleuchtende Fläche insofern problematisch, weil dabei

Rp 5 Kli / 15.8.1978

0001403

einerseits die Schattenarmut nicht nachteilig verschlechtert und andererseits die Farbtemperatur des Lichtes nicht verändert werden darf.

Die Helligkeit des Lichtflecks durch Änderung der Lampenspannung zu ändern, wie es bei bekannten solchen Beleuchtungsvorrichtungen gemacht wird, hat den Nachteil, daß sich die Farbtemperatur des Lichtfleckes dabei verändert.

Aus dem Bereich der Fototechnik ist es zwar bei Belichtungslampen bekannt (DE-GM 70 01 854), den Reflektor durch zwei schwenkbare Teilmantelflächen in einen hinteren und vorderen Teil zu unterteilen, wobei im hinteren Teil die Lichtquelle liegt und der vordere Teil die Lichtaustrittsöffnung enthält, und die beiden Teilmantelflächen über seitlich angeordnete Sektorstücke mit einem hinter dem Reflektor befindlichen Getriebe zu verbinden, so daß sie in den Strahlengang des Lichtes zwischen Lampe und Lichtaustrittsstelle, und zwar von außen nach innen zur Längsachse hin geschoben werden können. Eine solche Anordnung zur Helligkeitsregelung bei Beleuchtungsvorrichtungen der eingangs genannten Gattung mit einem relativ eng begrenzten Lichtfleck vorzusehen, würde jedoch zu einer Verschlechterung der Schattenarmut und durch innere Reflexion an der Rückseite der Teilmantelflächen zu einer Verwaschung der Hell-Dunkel-Grenze des an sich relativ scharfen Lichtfleckes führen, wodurch die Blendgefahr insbesondere bei Ausleuchtung der Mundhöhle eines Patienten erhöht wird. Die schwenkbaren Teilmantelflächen benötigen außerdem relativ viel Platz, wodurch das Leuchtengehäuse unnötig groß wird.

Der Erfindung liegt die Aufgabe zugrunde, eine Beleuchtungsvorrichtung der eingangs genannten Art dahingehend zu verbessern, daß eine Helligkeitsänderung unter Beibehaltung der Lichtfleckgröße und des an sich relativ scharf begrenzten Lichtfleckes bei gleichbleibender Farbtemperatur und Erhaltung der Schattenarmut möglich ist. Die Gehäuseabmessungen sollen sich durch die vorzuschlagenden Maßnahmen nicht vergrößern.

Die gestellte Aufgabe wird bei einer Beleuchtungsvorrichtung der eingangs genannten Art gemäß der Erfindung dadurch gelöst, daß im unmittelbaren Bereich der Lampe zwischen dieser und dem Reflektor im Strahlengang des Lichtes eine die Strahlen wenigstens teilweise abschirmende Blende angeordnet ist, und die Blende im Sinne einer Abschirmung der Strahlen vom Zentrum zur Peripherie des Reflektors hin verstellbar angeordnet ist. Durch das Einführen der Blende zwischen Lampe und Reflektor im unmittelbaren Bereich der Lampe läßt sich die wirksame Reflektorfläche entsprechend der gewünschten Helligkeitsänderung unter Beibehaltung der Farbtemperatur und der Schattenarmut verändern, wobei die Lichtfleckgröße und die an sich scharfe Begrenzung des Lichtflecks erhalten bleiben. Da die Lampenspannung nicht verändert wird, bleibt auch die Farbtemperatur über den gesamten Regelbereich erhalten.

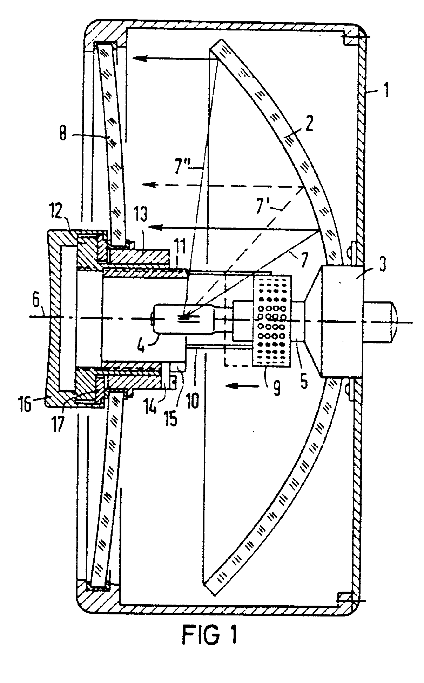

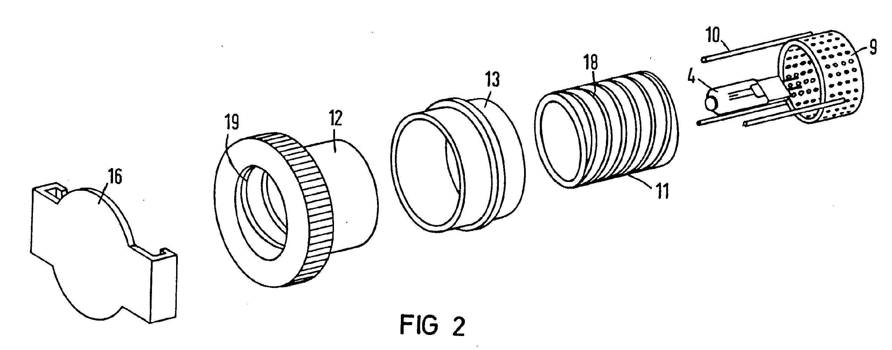

Vorteilhafte Weiterbildungen der Erfindung sind in den Unteransprüchen enthalten. Ein Ausführungsbeispiel der Erfindung wird anhand der Figuren 1 und 2 näher erläutert. Es zeigen:

Fig. 1 ein Ausführungsbeispiel einer zahnärztlichen Beleuchtungsvorrichtung nach der Erfindung im Längsschnitt,

Fig. 2 Einzelheiten der Vorrichtung nach Fig. 1.

Die Fig. 1 zeigt eine zahnärztliche Beleuchtungsvorrichtung mit einem Gehäuse 1, in dessen Innerem ein Reflektor 2 mittels eines entsprechend ausgebildeten Halteteils 3 gehaltert ist. Der Reflektor 2 kann Teil eines Ellipsoids oder eines Paraboloids sein. Etwa im inneren Brennpunkt des Reflektors 2 ist eine Lampe 4 mit Hilfe eines Sockels 5 gehaltert. Der Sockel 5 ist im Halteteil 3 längs der Lampenachse 6 verschiebbar gehaltert, so daß durch Verschieben der Lampe relativ zum inneren Brennpunkt die Größe des außerhalb des Reflektors (in der Regel in einem Abstand von 80 bis 100 cm) erzeugten Lichtfleckes optimal eingestellt werden kann. Nach erfolgter Einstellung des Lichtfleckes bleibt der Abstand Lampe - Reflektor erhalten. Die von der Lampe 4 ausgehenden Lichtstrahlen (7, 7', 7'') werden vom Reflektor 2 so reflektiert, daß sie nach Durchgang durch eine Streuscheibe 8 in dem genannten Abstand von etwa 80 bis 100 cm einen Lichtfleck von etwa 100 x 200 mm erzeugen.

Mit 9 ist eine über ihren Umfang mit Durchbrechungen versehene hülsenförmige Blende bezeichnet, die mittels mehrerer Abstandsglieder 10 an einem Abschirmrohr 11 befestigt ist. Das Abschirmrohr 11 umgibt die Lampe 4 konzentrisch, und zwar derart, daß die vom Lampenfaden ausgehenden Lichtstrahlen (7'') gerade noch auf den Reflektor 2 auftreffen, also keine unerwünschten Reflexionen im Lampengehäuse hervorrufen können. Mit dem Abschirmrohr 11 ist eine als Verstellglied dienende Hülse 12 verbunden, welche in einer Führungshülse 13 drehbar geführt ist. An der Führungshülse 13 ist ein Führungsstift 14 befestigt, der in eine entsprechend ausgebildete Nut 15 im Abschirmrohr 11 eingreift. Die äußere, also die vom Reflektor 2 wegweisende stirnseitige Öffnung der Hülse 12 ist gegen direkte Einsicht

0001403

durch ein Abschirmteil 16 abgedeckt. Das Abschirmteil ist mittels zweier abgewinkelter Endteile auf ein zwischen Streuscheibe 8 und Verstellglied 12 angeordnetes Halteteil 17 leicht lösbar aufgeschnappt. Das die Öffnung der Hülse 12 abdeckende Mittelteil des Abschirmteils 16 ist der besseren Abfuhr der von der Lampe erzeugten Wärme wegen mit einem entsprechend großen Abstand zur äußeren Stirnfläche der Hülse 12 angeordnet.

Die Fig. 2 zeigt in einer Explosionsdarstellung den Aufbau und die Anordnung der zur Abschirmung der Lichtstrahlen zwecks Änderung der Lichtintensität erforderlichen Bauteile. Aus der Darstellung ist zu entnehmen, daß das Abschirmrohr 11 mit einem Außengewinde 18 versehen ist, das mit einem entsprechenden Innengewinde 19 des Verstellgliedes 12 zusammenwirkt. Durch Drehen des Verstellgliedes 12 wird das über den Stift 14 gegen Verdrehung gesicherte Abblendrohr 11 axial verschoben.

Die hülsenförmige Blende 9 kann - wie dargestellt - entweder mit Durchbrechungen versehen sein oder auch aus einem teilweise transparenten Material bestehen, z.B. aus Opalglas.

In Fig. 1 ist die Blende 9 in ihrer äußeren Endstellung gezeichnet. In dieser Stellung fallen praktisch sämtliche Strahlen von der Lichtquelle 4 auf den Reflektor und werden von diesem zur Bildung des Lichtfleckes zurückreflektiert. Wird die Blende 9 in Pfeilrichtung bis in die gestrichelte Stellung nach links bewegt, was - nach Abnahme des Abschirmteils 16 - durch Drehen des Verstellgliedes 12 geschehen kann, so wird ein Teil der Strahlen, nämlich der zwischen den Strahlen 7 und 7', abgeschirmt. Wegen der Durchbrechungen in der Blende ist in diesem Bereich nur eine teilweise Abschirmung gegeben. Wenn eine stärkere Abschirmung erwünscht ist, kann ein zweckmäßigerweise innen schwarz gefärbtes Abschirmteil ohne Durchbrechungen verwendet werden. Bei einer weniger starken Abschirmung kann auch eine transparente Blende vorgesehen werden. In allen Fällen wird wegen der Anordnung der Blende im unmittelbaren Bereich der Lampe und wegen der Verstellung der Blende von innen nach außen, also vom Reflektorzentrum zur Reflektorperipherie, eine Helligkeitsänderung erzielt, ohne daß dabei die Lichtfleckgröße, die Schattenarmut sowie die Hell-Dunkel-Grenze an der Begrenzung des Lichtfleckes nachteilig verändert werden. Das Ausführungsbeispiel läßt erkennen, daß die Blende 9 von der Vorderseite der Leuchte aus verstellt wird. Denkbar ist es auch, die Konstruktion so auszuführen, daß die Blende von der Rückseite des Leuchtengehäuses aus verstellt werden kann.

0001403

Patentansprüche

1. Beleuchtungsvorrichtung für ärztliche, insbesondere zahnärztliche Zwecke, mit einem Reflektor und einer vorzugsweise in dessen innerem Brennpunkt angeordneten Lichtquelle, welche außerhalb des Reflektors, vorzugsweise in dessen äußerem Brennpunkt, einen relativ eng begrenzten Lichtfleck erzeugen, sowie mit Mitteln zur Änderung der Helligkeit des Lichtfleckes, d a d u r c h g e k e n n z e i c h n e t , daß im unmittelbaren Bereich der Lampe (4) zwischen dieser (4) und dem Reflektor (2) im Strahlengang des Lichtes eine die Strahlen wenigstens teilweise abschirmende Blende (9) angeordnet ist, und die Blende (9) im Sinne einer Abschirmung der Strahlen vom Zentrum zur Peripherie des Reflektors (2) hin verstellbar angeordnet ist.

2. Beleuchtungsvorrichtung nach Anspruch 1, d a - d u r c h g e k e n n z e i c h n e t , daß als Blende eine die Lampe (4) konzentrisch umgebende und in Richtung der Lampenachse (6) verstellbar gehalterte Hülse (9) vorgesehen ist.

3. Beleuchtungsvorrichtung nach Anspruch 2, d a - d u r c h g e k e n n z e i c h n e t , daß die Verstellung der Hülse (9) von der der Lampe (4) zugewandten Seite des Reflektors (2) aus erfolgt.

4. Beleuchtungsvorrichtung nach Anspruch 2, d a - d u r c h g e k e n n z e i c h n e t , daß die Hülse (9) mittels Abstandshalter (10) auf einem die Lampe (4) umgebenden Abschirmrohr (11) befestigt ist und das Abschirmrohr (11) in einer Führungshülse (13) axial bewegbar gehaltert ist.

5. Beleuchtungsvorrichtung nach Anspruch 4, d a -
d u r c h   g e k e n n z e i c h n e t , daß das
Abschirmrohr (11) mit einem Außengewinde (18) versehen
ist, das in Eingriff steht mit dem Innengewinde (19),
einer als Stellglied für die Blende (9) dienenden weiteren Hülse (12) und das Abschirmrohr (11) eine parallel zur Lampenachse (6) verlaufende Nut (15) enthält, in die ein mit der Führungshülse (13) verbundenes Führungsteil (14) eingreift.

6. Beleuchtungsvorrichtung nach Anspruch 5, d a -
d u r c h   g e k e n n z e i c h n e t , daß die
Hülse (12) stirnseitig von einem diese gegen axiales
Herausgleiten aus der Führungshülse (13) sichernden
Abschlußteil (16) umgeben ist.

7. Beleuchtungsvorrichtung nach Anspruch 6, d a -
d u r c h   g e k e n n z e i c h n e t , daß das
Abschlußteil (16) die äußere stirnseitige Öffnung
der Hülse (12) bedeckt und mit abgewinkelten Endteilen an einem mit dem Reflektor (8) verbundenen Halteteil (17) leicht abnehmbar gehaltert ist.

8. Beleuchtungsvorrichtung nach Anspruch 6 oder 7,
d a d u r c h   g e k e n n z e i c h n e t , daß
das Abschlußteil (16) mit einem Abstand zur äußeren
Stirnfläche der Hülse (12) angeordnet ist.

9. Beleuchtungsvorrichtung nach einem der Ansprüche
2 bis 8, d a d u r c h   g e k e n n z e i c h n e t ,
daß die Hülse (9) aus einem die Lichtstrahlen absorbierenden Material besteht.

000140

- 3 -      VPA 77 P 5116 EPC

10. Beleuchtungsvorrichtung nach einem der Ansprüche 2 bis 8, d a d u r c h   g e k e n n z e i c h n e t, daß die Hülse (9) aus einem das Licht teilweise durchlassenden, vorzugsweise durchscheinenden, Material besteht.

FIG 1

FIG 2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>US - A - 2 297 781</u> (KORENGOLD) | 1,2,7,9 |
| | * Seite 2, Spalte 2, Zeilen 16-39; Seite 3, Spalte 1, Zeilen 15-29; Figuren 5,6 * | |
| | -- | |
| | <u>DE - C - 1 042 496</u> (BLIN) | 1-4,6, 10 |
| | * Patentansprüche 1-3; Figur 5 * | |
| | -- | |
| | <u>FR - A - 1 311 965</u> (BLIN) | 1,2 |
| | * Seite 2, Spalte 1, Zeilen 10-19; Figur 1 * | |
| | -- | |
| | <u>GB - A - 327 563</u> (THE GENERAL ELECTRIC) | 3,4 |
| | * Figur 3 * | |
| | -- | |
| | <u>GB - A - 759 923</u> (TREHAWK MANUFAC- TURING) | 3,5,6 |
| | * Seite 2, Zeilen 5-19 * | |
| | ---- | |

**KLASSIFIKATION DER ANMELDUNG (Int.Cl.²)**

A 61 B 1/06
F 21 M 1/00

**RECHERCHIERTE SACHGEBIETE (Int. Cl.²)**

A 61 B
F 21 L
F 21 M
F 21 V

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent- familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 19-12-1978 | FOUCRAY |

EPA form 1503.1  06.78